# EUROPÄISCHE PATENTANMELDUNG

(11) **EP 4 721 657 A1**
(43) Veröffentlichungstag der Anmeldung: **08.04.2026**
(21) Anmeldenummer: 24205121.7
(22) Anmeldetag: 07.10.2024
(51) Int. Cl.: A61B 5/08, A61B 5/097, A61B 5/00, G01N 33/497

(54) **NICHT-INVASIVES VERFAHREN ZUR BESTIMMUNG VON ATEMGASEN AUFWEISEND SCHWEFELWASSERSTOFF**

(71) Anmelder: VOC - Advanced Breath Diagnostics GmbH, 91052 Erlangen (DE)
(72) Erfinder: VOIGT, Wieland, 91096 Möhrendorf (DE); MEKONNEN, Tessema Fenta, 91301 Forchheim (DE)
(74) Vertreter: Simandi, Claus

(57) **Zusammenfassung**

Die vorliegende Erfindung betrifft ein nicht-invasives Verfahren zur Bestimmung von Atemgasen aufweisend Schwefelwasserstoff, insbesondere zur Verwendung in der Medizin, Diagnose, Prädiktion, Risikostratifizierung und Therapiesteuerung von Erkrankungen, insbesondere Darmerkrankungen an Probanden, wobei durch Bakterien gebildete Gase in dem Ausatemgas bestimmt werden, wobei der Schwefelwasserstoff nicht nachteilig abgebaut wird.

## Beschreibung

Die vorliegende Erfindung betrifft ein nicht-invasives Verfahren zur Bestimmung von Atemgasen aufweisend Schwefelwasserstoff, insbesondere zur Verwendung in der Medizin, Diagnose, Prädiktion, Risikostratifizierung und Therapiesteuerung von Erkrankungen, insbesondere Darmerkrankungen an Probanden, wobei durch Bakterien gebildete Gase in dem Ausatemgas bestimmt werden, wobei der Schwefelwasserstoff nicht nachteilig abgebaut wird.

Die Bestimmung von Atemgasen, wie H₂, CH₄, CO₂ oder Schwefelwasserstoff (H₂S) aus Ausatemproben erfolgt in der klinischen Praxis entweder über Gaschromatographie oder Gassensoren. Entsprechende Teste sind kommerziell verfügbar und in der Literatur beschrieben (Gasbarrini A, Corazza GR, Gasbarrini G, Montalto M, Di Stefano M, Basilisco G, Parodi A, Usai-Satta P, Vernia P, Anania C, Astegiano M, Barbara G, Benini L, Bonazzi P, Capurso G, Certo M, Colecchia A, Cuoco L, Di Sario A, Festi D, Lauritano C, Miceli E, Nardone G, Perri F, Portincasa P, Risicato R, Sorge M, Tursi A; 1st Rome H2-Breath Testing Consensus Conference Working Group. Methodology and indications of H2-breath testing in gastrointestinal diseases: the Rome Consensus Conference. Aliment Pharmacol Ther. 2009 Mar 30;29 Suppl 1:1-49.).

Atemgasanalytik ("breath gas analysis") ist ein medizinisches Verfahren, bei dem die chemische Zusammensetzung der ausgeatmeten Luft analysiert wird, um Informationen über den Gesundheitszustand einer Person zu gewinnen. Diese Methode kann zur Diagnose von Krankheiten, zur Überwachung von Stoffwechselvorgängen, insbesondere in der Sportmedizin, Altersmedizin oder zur Analyse von Medikamenteneffekten sowie zur Therapiekontrolle eingesetzt werden.

Der Atemtest ist eine nicht-invasive Methode und kann z.B. zur Diagnose von "Small Intestinal Bacterial Overgrowth" (kurz: SIBO) wie folgt erläutert werden.

Vorbereitung: In den Tagen vor der Durchführung des SIBO-Tests wird der Proband gebeten, bestimmte Nahrungsmittel und Medikamente im Sinne einer Diät zu vermeiden, die das Testergebnis beeinflussen können. Dazu gehören oft Antibiotika, Laxantienten, Prokinetika und bestimmte ballaststoffreiche Lebensmittel.

Testsubstanz: Der Proband erhält eine Testsubstanz, die normalerweise Laktulose oder Glukose ist. Diese Substanzen sind vorzugsweise Kohlenhydrate, die normalerweise im Dünndarm nicht verdaut werden, aber von Darmbakterien fermentiert werden können, und eine Gasentwicklung provozieren.

Atemprobenentnahme: Der Proband atmet in spezielle Atembeutel oder Röhrchen bzw. Probenbehälter, die Atemgase aufnehmen können.

Messungen/Bestimmungen: Nachdem die Testsubstanz eingenommen wurde, werden in regelmäßigen Abständen Atemproben entnommen, normalerweise alle 15-30 Minuten über einen Zeitraum von einigen Stunden. Diese Proben werden dann auf ihren Gehalt an Atemgasen untersucht, die von den Darmbakterien produziert werden, wenn sie die Testsubstanz fermentieren. Die Atemgase werden detektiert z.B. mittels Gaschromatographie oder Massenspektrometrie.

Interpretation der Ergebnisse: Ein Anstieg der Atemgase im Atem deutet auf eine bakterielle Überwucherung im Dünndarm hin. Die Muster der Ausatemgase können auch helfen, zwischen verschiedenen Arten von SIBO zu unterscheiden.

Der Erfolg der Atemtests hängt jedoch von der genauen Menge und Verhältnis der Atemgase ab.

Schwefelwasserstoff (H₂S) spielt zunehmend eine wichtige Rolle in der Atemgasanalytik, insbesondere bei der Diagnose und Untersuchung von gastrointestinalen Erkrankungen, allerdings ist die Quantifizierung des Analyten herausfordernd. H₂S ist ein Gas, das von bestimmten Bakterien im Darm produziert wird, insbesondere während der Zersetzung von schwefelhaltigen Aminosäuren. Es kann im Atem nachgewiesen werden und liefert wertvolle Hinweise auf den Zustand des Verdauungstrakts und den bakteriellen Stoffwechsel.

Überraschenderweise wurde nunmehr gefunden, dass Schwefelwasserstoff (H₂S) in Gegenwart von Luftfeuchtigkeit, als Bestandteil der Atemgase, nicht stabil ist und abgebaut wird. Folglich können die Verhältnisse, Menge, Konzentration oder Quantifizierung von Schwefelwasserstoff verfälscht werden, insbesondere wenn nach Probenentnahmen das Atemgas in einem Probenbehälter verweilt.

Daher ist eine Aufgabe der vorliegenden Erfindung, die Bereitstellung eines Atemtests, sodass Probanden einen zuverlässigen Wert an Schwefelwasserstoff erhalten und besonders vorteilhaft die diagnostische Wertigkeit und Bestimmung verbessert wird.

Daher betrifft die Erfindung ein nicht-invasives Verfahren zur Bestimmung von Atemgasen aufweisend Schwefelwasserstoff, wobei durch Bakterien, insbesondere Darmbakterien, gebildete Gase in dem Ausatemgas eines Probanden bestimmt werden, wobei
i.) die enthaltende Feuchtigkeit des Ausatemgases durch Trocknung entzogen wird,
   und / oder
ii.) der enthaltende Schwefelwasserstoff des Ausatemgases durch nicht-adsorbierende inerte Materialien geführt und / oder gespeichert wird.

Weiterhin betrifft die Erfindung in einer weiteren bevorzugten Ausführungsform ein vorstehendes nicht-invasives Verfahren, wobei die Trocknung durch i.) Kühlung oder durch ii) ein Trocknungsmittel erfolgt, insbesondere mittels Calciumchlorid.

Weiterhin betrifft die Erfindung in einer weiteren bevorzugten Ausführungsform ein nicht-invasives Verfahren, wobei die Trocknung durch Kühlen mittels einer Kühlfalle erfolgt. Beispielsweise kann die Kühlfalle Teil eines Probenröhrchens sein oder zum Probenraum angeordnet sein, so dass die Luftfeuchtigkeit dem Ausatemgas entzogen wird. Dies erfolgt üblicherweise bei Temperaturen von weniger als 0 Grad Celsius.

Weiterhin betrifft die Erfindung in einer weiteren bevorzugten Ausführungsform nicht-adsorbierende inerte Materialien, die den Schwefelwasserstoff nicht adsorbieren und auf diese Weise der Schwefelwasserstoff nicht aus der Atemgasprobe entfernt wird. Das nicht-adsorbierende inerte Material kann Bestandteil des Probenbehälters sein, insbesondere in Form der Wand des Probenbehälters. Weiterhin können Leitungen oder Röhren zur Führung des Atemgases einschließlich des Mundstückes bzw. Aufnahmeeinheit aus einem solchen nicht-adsorbierenden inerten Material bestehen. Dem Fachmann sind solche nicht-adsorbierende inerte Materialien bekannt.

Nicht-adsorbierende inerte Materialien sind Substanzen, die keine nennenswerte Wechselwirkung mit anderen Stoffen eingehen, insbesondere nicht durch Adsorption, und chemisch reaktionsträge sind.

"Inert" bedeutet, dass das Material chemisch stabil ist und sich nicht leicht mit anderen Stoffen verbindet.

"Nicht-adsorbierend" bedeutet, dass das Material keine Teilchen aus der Umgebung an seiner Oberfläche festhält.

Insbesondere können siliziumhaltige Materialien mit Schwefelwasserstoff in Wechselwirkung treten.

Zum Beispiel sind übliche Gläser nicht inert. Sie können Metallionen abgeben, die katalytisch Abbaureaktionen des Schwefelwasserstoffs auslösen können. Ebenfalls können Fehlstellen der Oberfläche chemische Reaktionen katalysieren und als Keimstelle für Kristallisation dienen.

Eine Silikonisierung von Gläsern oder anderen Materialien kann eine erforderliche Inertheit herstellen und eine Adsorption verhindern.

Eine Silikonschicht kann temporär (Befilmung) oder dauerhaft mittels Einbrennsilikonisierung oder Beschichtungsverfahren, wie die chemische Gasphasenabscheidung (chemical vapour deposition, CVD) auf eine Glasoberfläche oder andere Materialoberfläche aufgebracht werden. Die unterschiedlichen Herstellungsmethoden sind dem Fachmann bekannt (Diss. T. Mundry, Humboldt-Universität, Berlin, 1999).

Die Einbrennsilikonisierung liefert zumeist eine homogene, ca. 15 - 50 nm dünne hydrophobe Schicht und deckt die initial vorhandene Glasoberfläche oder andere Materialoberfläche und vorhandene Fehlstellen ab (Reuter B., Petersen C., Die Silikonisierung von Spritzen, TechnoPharm2, Nr.4, 238-244 (2012)). Infolgedessen wird eine reduzierte Wechselwirkung zwischen Schwefelwasserstoff und dem silikonisierten Material erreicht.

Geeignete nicht-adsorbierende inerte Materialien sind weiterhin solche wie hydrierte amorphe Siliziumsubstrate (z.B. SilcoNert^{®} 2000 (Bellefonte, USA), wie in US 6,444,326 B1 beschrieben, Quarzglas, u.a. Beschichtungen.

Weiterhin können Kunststoffe, die nicht-adsorbierende Eigenschaften aufweisen, verwendet werden, wie Polytetrafluorethylen (PTFE), Polyethylen (PE), Polypropylen (PP), Polyvinylchlorid (PVC), Polystyrol (PS), Polyethylenterephthalat (PET) oder Polymethylmethacrylat (PMMA) .

Daher betrifft die Erfindung ebenfalls das erfindungsgemäße Verfahren, wobei der enthaltende Schwefelwasserstoff des Ausatemgases durch nicht-adsorbierende inerte Materialien geführt und / oder gespeichert wird.

Die Führung des Atemgases kann über das Mundstück bzw. Aufnahmeeinheit in Leitungen und Röhren erfolgen, zum einen in den Probenbehälter, ggfs. mit einer angeordneten Kühlfalle oder zum Führen in den Detektor. Die Speicherung des Atemgases kann in einem Probenbehälter erfolgen. Der Probenbehälter zur Aufnahme der Atemgase als solcher, insbesondere in Form der Wand des Probenbehälters können aus einem nicht-adsorbierenden inerten Material bestehen. Weiterhin können Leitungen oder Röhren zur Führung des Atemgases einschließlich des Mundstückes bzw. Aufnahmeeinheit aus einem solchen nicht-adsorbierenden inerten Material bestehen. Dem Fachmann sind solche nicht-adsorbierende inerte Materialien bekannt.

Besonders vorteilhaft ist, dass durch das erfindungsgemäße Verfahren der Gehalt von anderen Atemgasen nicht beeinträchtigt wird. Folglich kann mithilfe des erfindungsgemäßen Verfahrens, erstmals eine zuverlässige Kalibrierung auf Basis von Schwefelwasserstoff erfolgen.

In einer weiteren Ausführungsform betrifft die Erfindung ein nicht-invasives Verfahren zur Bestimmung von Atemgasen aufweisend Schwefelwasserstoff zur Verwendung in der Medizin, insbesondere Sportmedizin oder Altersmedizin, einschließlich der Überwachung von Stoffwechselvorgängen oder Diagnose oder Prädiktion oder zur Risikostratifizierung oder Therapiesteuerung von Erkrankungen von Probanden.

Im Sinne dieser Erfindung sind Erkrankungen wie Gastrointestinale Erkrankungen, insbesondere Darmerkrankungen, SIBO (Small Intestinal Bacterial Overgrowth), Reizdarmsyndrom (IBS), Laktose- oder Fruktosemalabsorption, chronisch entzündliche Darmerkrankungen (CED) (Guo F-F, Yu T-C, Hong J and Fang J-Y (2016) Emerging Roles of Hydrogen Sulfide in Inflammatory and Neoplastic Colonic Diseases. Front. Physiol. 7:156), insbesondere Morbus Crohn und Colitis ulcerosa, Zöliakie oder funktionelle Dyspepsie. Insbesondere sind solche Darmerkrankungen erfindungsgemäß umfasst, die eine Malabsorption aufweisen. Weiterhin sind erfindungsgemäß an Erkrankungen solche umfasst ausgewählt aus der Gruppe Krebserkrankungen, insbesondere das Kolonkarzinom (Du P, Tseng Y,Liu P, et al. Role of exhaled hydrogen sulfide in the diagnosis of colorectal cancer, BMJ Open Gastroenterol 2024,11), sowie kardiovaskuläre Erkrankungen, insbesondere arteriosklerotischer Art (ASCVD) sowie Hauterkrankungen, insbesondere Wundheilung und Psoriasis (Xiao Q, Xiong L, Tang J, Li L, Li L, Hydrogen Sulfide in Skin Diseases: A Novel Mediator and Therapeutic Target. Oxid Med Cell Longev. 2021 Apr 20;2021:6652086). Darüber hinaus ist auch der Nachweis von Mundgeruch (Halitosis) im Sinne dieser Erfindung umfasst. Weiterhin relevante Erkrankungen im Sinne dieser Erfindung sind Lungenerkrankungen und Erkrankungen der Atemwege, insbesondere Asthma und COPD (Debraj Jash , Hydrogen sulphide as a biomarker in COPD and asthma, European Respiratory Journal 2014,44), neurologische Erkrankungen, insbesondere Alzheimer und andere Formen der Demenz.

Die Bakterien können z.B. durch Infektionen vorgenannte Erkrankungen beeinflussen oder sind gar ursächlich hierfür. Weiterhin können eine oder mehrere vorgenannte Erkrankungen umfasst sein.

Die Bakterien können insbesondere Darmbakterien sein, oder am Ort der Besiedelung ausgewiesen werden, wie Magen, Lunge, Atemwege u.a.

Eine weitere Aufgabe der Erfindung ist ebenfalls die Therapiesteuerung mittels einer Prädiktion oder Diagnose von Erkrankungen, insbesondere Darmerkrankungen, wobei ein Proband nach einem erfindungsgemäßen Verfahren untersucht wird, mit der Maßgabe, dass der Proband Medikamente, insbesondere Antibiotika/Phytobiotika zur Behandlung einer Erkrankung, insbesondere Darmerkrankung erhält oder eine Diät zur Behandlung einer Darmerkrankung einhält, insbesondere zur Behandlung einer Malabsorption, sodass ein Therapieerfolg oder Therapieverbesserung erfolgen kann.

"Diagnose" oder "Prädiktion" im Sinne dieser Erfindung bedeutet die positive Feststellung oder Vorhersage / Prognose bzw. Wahrscheinlichkeit des Ein- und Auftretens einer Erkrankung, insbesondere Darmerkrankung.

Der Begriff der Diagnose umfasst weiterhin die medizinische Diagnostik und diesbezügliche Untersuchungen, insbesondere die in-vitro Diagnostik und Labordiagnostik.

Ferner betrifft die Erfindung ein Verfahren zum Stratifizieren, insbesondere zur Risikostratifizierung und / oder Therapiesteuerung eines Patienten.

"Stratifizieren oder Therapiesteuerung" im Sinne dieser Erfindung bedeutet, dass das erfindungsgemäße Verfahren Entscheidungen zur Behandlung und Therapie des Probanden erlauben, sei es Hospitalisierung des Patienten, Einsatz, Wirkung und / oder Dosierung eines oder mehrerer Arzneimittel, eine therapeutische Maßnahme oder die Überwachung eines Krankheitsverlaufs sowie des Therapieverlaufs bzw. Ätiologie oder Klassifizierung von Erkrankungen.

In einer weiteren Ausführungsform der Erfindung umfasst der Begriff "Stratifizierung" insbesondere die Risikostratifizierung mit der Prädiktion eines "outcome" eines nachteiligen gesundheitlichen Ereignisses, insbesondere, dass eine Erkrankung vorliegt.

Im Rahmen dieser Erfindung wird unter "Proband" ein beliebiger Mensch oder Säugetier verstanden, insbesondere ein Patient. Besonders bevorzugt sind Patienten, die bereits Symptome aufweisen, und zwar Blähungen, Bauchschmerzen, Durchfall, Verstopfung, Nährstoffmangel und Gewichtsverlust aufgrund Malabsorption.

Weiterhin betrifft die Erfindung die Verwendung einer Vorrichtung zur Bestimmung von Atemgasen aufweisend Schwefelwasserstoff, wobei durch Bakterien, insbesondere Darmbakterien, gebildete Gase in dem Ausatemgas eines Probanden bestimmt werden, wobei die Vorrichtung Mittel umfasst,
i.) dass die enthaltende Feuchtigkeit des Ausatemgases durch Trocknung entzogen wird,
   und / oder
ii.) der enthaltende Schwefelwasserstoff des Ausatemgases durch nicht-adsorbierende inerte Materialien geführt und / oder gespeichert wird.

Die Vorrichtung weist ein Mundstück bzw. Aufnahmeeinheit auf, wobei die Führung des Atemgases in Leitungen und Röhren erfolgen, zum einen in den Probenbehälter, ggfs. mit einer angeordneten Kühlfalle oder über ein Trockenmittel und ggfs. zum Führen in einen Detektor. Die Speicherung des Atemgases kann in einem Probenbehälter erfolgen. Der Probenbehälter zur Aufnahme der Atemgase als solcher, insbesondere in Form der Wand des Probenbehälters bestehen aus einem nicht-adsorbierenden inerten Material. Weiterhin können Leitungen oder Röhren zur Führung des Atemgases einschließlich des Mundstückes aus einem solchen nicht-adsorbierenden inerten Material bestehen.

Eine solche Vorrichtung besteht weiterhin aus einem geeigneten Detektionsmittel zur Detektion der Atemgase (z.B. Flammenphotometerdetektor) zur erfindungsgemäßen Verwendung.

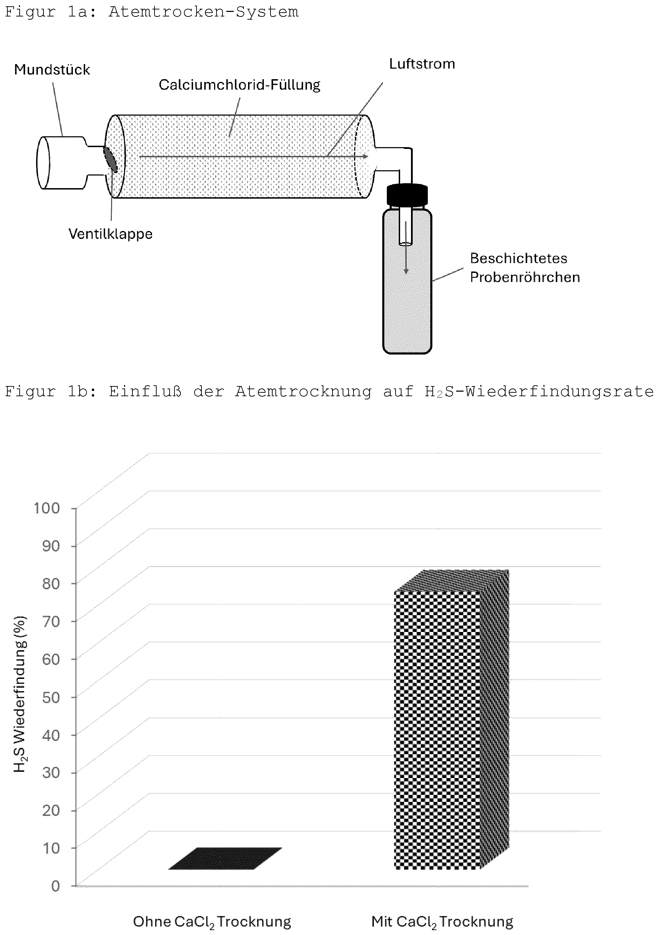

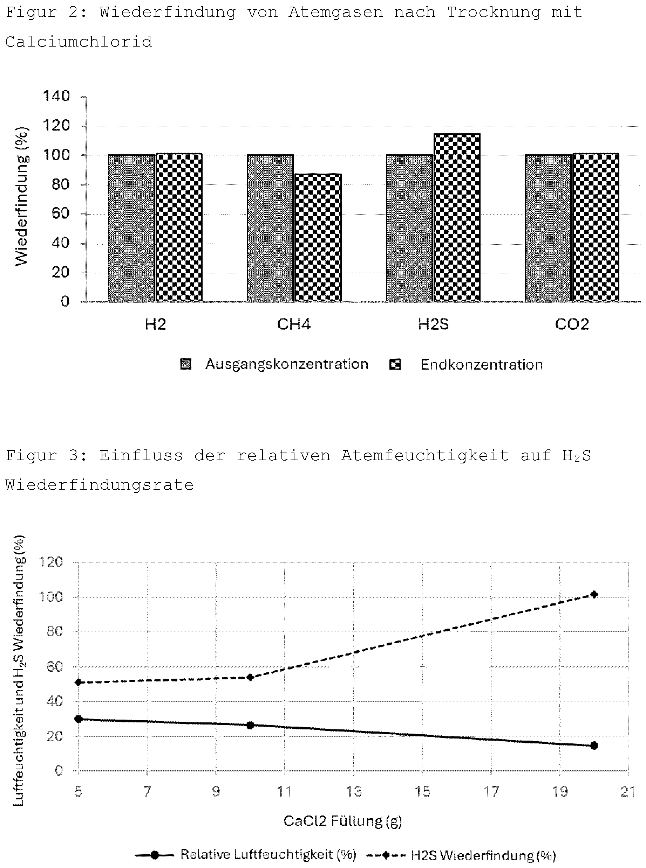

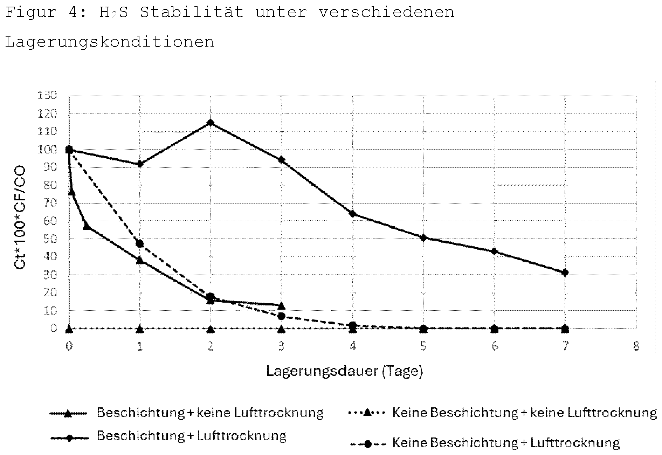

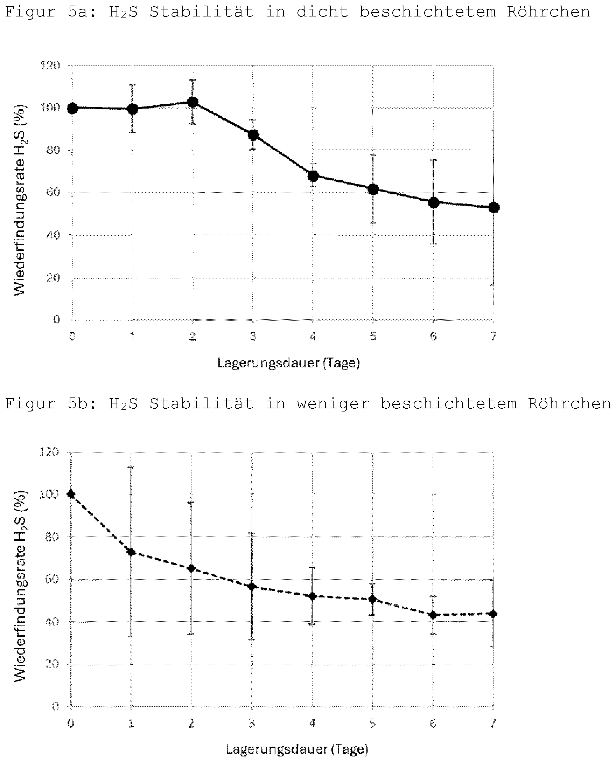

Eine beispielhafte Vorrichtung ist in Figur 1a abgebildet, wobei die Trocknung über ein Trockenmittel erfolgt.

Es sei darauf hingewiesen, dass Merkmale, die im Zusammenhang mit einer beispielhaften Ausführungsform oder einem beispielhaften Gegenstand beschrieben werden, mit jeder anderen beispielhaften Ausführungsform oder mit jedem anderen beispielhaften Gegenstand kombiniert werden können.

Wenn ein Begriff mit einem unbestimmten oder bestimmten Artikel, wie zum Beispiel "ein" im Singular bezeichnet wird, schließt dies auch den Begriff im Plural mit ein und umgekehrt, sofern der Kontext nicht eindeutig anderes festlegt.

Der Ausdruck "umfassen", wie er hier verwendet wird, schließt nicht nur die Bedeutung von "enthalten" ein, sondern kann auch "bestehen(d) aus" und "im Wesentlichen bestehend aus" bedeuten.

Die Erfindung wird nun in den folgenden Beispielen und Figuren näher beschrieben, ohne darauf beschränkt zu sein.

### Beispiele:

### Beispiel 1:

### Experimentelle Anordnungen

Messgerät: Proben/Standards wurden mit Trockenröhrchen und Headspace-Glasröhrchen gesammelt. Die Messungen wurden mit einem Gaschromatographen mit Flammenphotometerdetektor (GC-FPD) durchgeführt, der mit einer SH-U-Bond-Säule (30 m x 0,32 mm und 10 µm Filmdicke) und einem AOC-6000 Plus-Autosampler (Shimadzu, Duisburg, Deutschland) ausgestattet war. Der Wasserstoff (99 % Reinheit) wurde mit einem HG BASIC 180-Generator (LNI Swissgas GmbH, Kamen, Deutschland) erzeugt. Die Säulentemperatur wurde isotherm bei 65 0C gehalten. Die FPD- und Injektionstemperatur wurden bei 200 0C bzw. 250 0C gehalten. Der Durchfluss von synthetischer Luft (von Air Liquid, Regensburg, Deutschland) und Wasserstoff betrug 60 ml/min bzw. 40 ml/min. Das Injektionsvolumen und der Durchfluss des Helium-Trägergases (Reinheit 99,9999 %) betrugen 500 µl bzw. 4 ml/min.

Drying tube (Trocknungsrohr):
- Das Trocknungsröhrchen wurde aus einem Polyethylenröhrchen hergestellt, indem es mit 20 g CaCl2-Granulat (Carl Roth GmbH+Co.KG, Karlsruhe, Deutschland) gefüllt und mit einem Polyethylenmundstück verbunden wurde.

Zur Herstellung von Schwefelwasserstoff-Standards:
- 1 1 Polypropylen-Tedlar-Atembeutel von SKC Inc. (Kat.-Nr.: 249-01-PP, Pennsylvania, USA).
- 3 ppm Schwefelwasserstoff-Standard in N₂ von All-In-Gas (Teilecode: C020512, Starnberg, Deutschland).

Beschichtung von Glasfläschchen:
- 20-mL-Headspace-Fläschchen (Carl Roth GmbH+Co.KG, Karlsruhe, Deutschland)
- SilcoNert^{®} 2000 von SilcoTek GmbH (Bad Homburg, Deutschland).

### Figuren:

Figur 1:
   a) Darstellung des Aufbaus des Atemtrockenröhrchens mit Auslass der Atemluft in ein beschichtetes Probenröhrchen.
   b) Der ausgeatmete Atem strömt durch ein CaCl₂-Rohr in ein unbeschichtetes Probenröhrchen; dann wird H₂S hinzugefügt (C_{spiked} = 143 ppb). Trocknung der Atemluft führt zur Steigerung der H₂S Wiederfindungsrate.
Figur 2:
   Ein Gemisch aus vier Gasen (Wasserstoff, Methan, Kohlendioxid und Schwefelwasserstoff) wurden in einem Ausatembeutel vorbereitet und die Konzentration jedes Gases wurde aus dem Ausatembeutel gemessen (C₀= 30 ppm H₂, 9 ppm CH₄, 3 % CO₂, 350 ppb H2S) und nach dem Durchgang durch ein mit CaCl₂ gefülltes Rohr (C). Die Trocknungsmethode führt zu keinem Verlust der genannten Atemgase.
Figur 3:
   300 ppb H₂S im ausgeatmeten Atem, der in einem Atembeutel aufgefangen wird. Die Feuchtigkeit im Beutel wird mit einem Hygrometer gemessen. Dann werden drei Trocknungsröhrchen mit 5, 10 und 20 g CaCl₂ vorbereitet. Das Gas wird durch die Trocknungsröhrchen geleitet und mit einem GC-FPD gemessen. Die Feuchtigkeit nach dem Durchgang durch die Röhrchen wird ebenfalls gemessen. Die Trocknung des Atems führt zu einer Steigerung der Wiederfindung von Schwefelwasserstoff auf 100%.
Figur 4:
   H₂S wird in ausgeatmeter Luft aufbereitet und in beschichteten und unbeschichteten Röhrchen mit/ohne Trocknungsröhrchen gesammelt. Die Konzentration wurde über einen Zeitraum von bis zu 7 Tagen täglich gemessen. Die Ergebnisse demonstrieren einen unzureichenden Effekt der Beschichtung alleine jedoch einen starken Effekt der Trocknung des Atems auf die Stabilität von H₂S in Atemproben.
Figur 5a und Figur 5b:
   1 ppm (Co) H2S in der ausgeatmeten Luft wurde in drei dicht beschichteten (undurchsichtigen) und drei weniger beschichteten (transparenten) Röhrchen durch Trocknungsröhrchen geleitet. Die Proben wurden an sieben aufeinanderfolgenden Tagen gemessen (Ct) und die Wiederfindung wurde wie folgt berechnet: (Ct/Co)*100. Die dargestellten Diagramme sind der Durchschnitt von drei Wiederholungen für jeden Tag mit den Fehlerbalken.

## Patentansprüche

1. Nicht-invasives Verfahren zur Bestimmung von Atemgasen aufweisend Schwefelwasserstoff, wobei durch Bakterien gebildete Gase in dem Ausatemgas eines Probanden bestimmt werden, **dadurch gekennzeichnet,**
i.) **dass** die enthaltende Feuchtigkeit des Ausatemgases durch Trocknung entzogen wird,
und / oder
ii.) der enthaltende Schwefelwasserstoff des Ausatemgases durch nicht-adsorbierende inerte Materialien geführt und / oder gespeichert wird.

2. Nicht-invasives Verfahren zur Bestimmung von Atemgasen aufweisend Schwefelwasserstoff nach Anspruch 1, wobei die Trocknung durch i.) Kühlung oder durch ii) ein Trocknungsmittel erfolgt, insbesondere mittels Calciumchlorid.

3. Nicht-invasives Verfahren zur Bestimmung von Atemgasen aufweisend Schwefelwasserstoff nach Anspruch 1, wobei die Trocknung durch Kühlen mittels einer Kühlfalle erfolgt.

4. Nicht-invasives Verfahren zur Bestimmung von Atemgasen aufweisend Schwefelwasserstoff nach Anspruch 1, wobei nicht-adsorbierende inerte Materialien ausgewählt sind aus der Gruppe der Silikonbeschichtungen, Kunststoffe, hydrierte amorphe Siliziumsubstrate und Quarzglas.

5. Nicht-invasives Verfahren zur Bestimmung von Atemgasen aufweisend Schwefelwasserstoff nach einem der vorstehenden Ansprüche zur Verwendung in der Medizin, insbesondere Sportmedizin oder Altersmedizin, einschließlich der Überwachung von Stoffwechselvorgängen.

6. Nicht-invasives Verfahren zur Bestimmung von Atemgasen aufweisend Schwefelwasserstoff nach einem der vorstehenden Ansprüche zur Verwendung in der Diagnose oder Prädiktion oder zur Risikostratifizierung oder Therapiesteuerung von Erkrankungen von Probanden.

7. Nicht-invasives Verfahren zur Bestimmung von Atemgasen aufweisend Schwefelwasserstoff nach einem der vorstehenden Ansprüche zur Verwendung in der Diagnose oder Prädiktion oder zur Risikostratifizierung oder Therapiesteuerung von Erkrankungen von Probanden, wobei eine oder mehrere Erkrankung ausgewählt ist aus der Gruppe gastrointestinale Erkrankungen, insbesondere Darmerkrankungen, solche wie SIBO (Small Intestinal Bacterial Overgrowth), Reizdarmsyndrom (IBS), Laktose- oder Fruktosemalabsorption, chronisch entzündliche Darmerkrankungen (CED), insbesondere Morbus Crohn und Colitis ulcerosa, Zöliakie oder funktionelle Dyspepsie; Krebserkrankungen, insbesondere Kolonkarzinom, Gefäßerkrankungen, insbesondere arteriosklerotischer Art (ASCVD),Hauterkrankungen, insbesondere Wundheilung und Psoriasis, Mundgeruch (Halitosis), Lungenerkrankungen, insbesondere Asthma und COPD, neurologische Erkrankungen, insbesondere Alzheimer und Formen der Demenz.

8. Nicht-invasives Verfahren zur Bestimmung von Atemgasen aufweisend Schwefelwasserstoff nach einem der vorstehenden Ansprüche zur Verwendung in der Therapiesteuerung, mit der Maßgabe, dass der Proband Medikamente zur Behandlung einer Erkrankung erhält oder eine Diät zur Behandlung einer Erkrankung einhält, insbesondere zur Behandlung einer Darmerkrankung oder Malabsorption.

9. Verwendung einer Vorrichtung zur Bestimmung von Atemgasen aufweisend Schwefelwasserstoff, wobei durch Bakterien gebildete Gase in dem Ausatemgas eines Probanden bestimmt werden, wobei die Vorrichtung Mittel umfasst, **dadurch gekennzeichnet,**
i.) **dass** die enthaltende Feuchtigkeit des Ausatemgases durch Trocknung entzogen wird,
und / oder
ii.) der enthaltende Schwefelwasserstoff des Ausatemgases durch nicht-adsorbierende inerte Materialien geführt und / oder gespeichert wird.
